# EUROPEAN PATENT APPLICATION

(11) **EP 1 598 075 A1**
(43) Date of publication of application: **23.11.2005**
(21) Application number: 04468008.0
(22) Date of filing: 21.05.2004
(51) Int. Cl.: A61K 38/18, C07K 14/505

(54) **Process for the isolation and / or purification of proteins**

(71) Applicant: LEK Pharmaceuticals d.d., 1526 Ljubljana (SI)
(72) Inventor: Menart, Viktor, 1370 Logatec (SI); Kenig, Maja, 1353 Borovnica (SI); Gaberc-Porekar, Vladka, 1000 Ljubljana (SI); Fonda, Irena, 1360 Vrhnika (SI)
(74) Representative: Dietz, Jörg-Reimar

(57) **Abstract**

The invention relates to the process for the isolation and / or purification of biologically active proteins, preferably TNF-alpha or TNF-alpha analogues. The process of the present invention results in the production of high yields of proteins, preferably TNF-alpha or TNF-alpha analogues with a purity of greater than 98%. The described process is particularly suitable for the industrial production of proteins, preferably TNF-alpha or TNF-alpha analogues.

## Description

### Field of the invention

The invention relates to a process for the isolation and / or purification of proteins in a highly pure form. This is achieved by use of a specific chromatographic matrix in a specific sequence of purification steps. The process of the present invention can be applied for a wide range of proteins, preferably for tumour necrosis factor - alpha (TNF-alpha) and TNF-alpha analogues.

### Background of the invention

In medicine the delivery of the proteins in a highly pure form is required. Therefore, it is important for today's pharmaceutical industry to deliver highly purified proteins with low level of impurities. One such process is described in the present invention. In addition, the process of the present invention is highly economical and efficient and may be used for industrial production of proteins, preferably for TNF-alpha and TNF-alpha analogues.
Human TNF-alpha is a protein, which belongs to the family of cytokines. It is an essential element in the cascade of factors, which are involved in cell immune response and is involved in the pathogenesis of different acute infections and chronic immune or inflammatory diseases. In general, it has a pleiotropic activity in both, healthy and unhealthy organism. TNF-alpha exhibits an extensive anti-tumour effect and is used in medicine as an anti-cancer agent in a local therapy. Due to its toxic nature causing side effects by systemic use many TNF-alpha analogues are designed and prepared what led to conserved or even increased anti-tumour activity with less side effects.

### Summary of the invention

It is an object of the invention to deliver a highly efficient process for the isolation and / or purification of proteins, preferably of TNF-alpha and TNF-alpha analogues, and to provide biologically active proteins, preferably TNF-alpha and TNF-alpha analogues in a purified and biologically active form, as well as a pharmaceutical composition comprising the same.

According to the present invention it was found that a high purity of proteins, preferably of TNF-alpha and TNF-alpha analogues, can be achieved by using an isolation and / or purification process which comprises an affinity chromatography step. Preferably, an affinity chromatography is performed on an affinity chromatography matrix to which a glycosaminoglycan, in particular heparin is bound. This step is essential for the high final purity of preferably TNF-alpha and TNF-alpha analogues and the same could apply for all proteins that bind to a glycosaminoglycan, e.g. heparin with greater affinity than the other residual proteins. Only two additional chromatographic steps, preferably an anion exchange chromatography and a size-exclusion chromatography, which are applied in a preferred embodiment of the present invention, can be used for achieving the high purity of the protein.
The process for the isolation and / or purification of proteins, preferably of TNF-alpha and TNF-alpha analogues, results in the production of biologically active proteins, preferably TNF-alpha and TNF-alpha analogues with a purity of greater than 98%.
The process is suitable for the production of large quantities of proteins, preferably TNF-alpha and TNF-alpha analogues and is suitable for the industrial production of proteins, preferably TNF-alpha and TNF-alpha analogues.

The present invention provides a process for the isolation and / or purification of proteins according to claim 1. Preferred embodiments are set forth in the dependent claims.

### Detailed description of the invention and preferred embodiments thereof

The present invention relates to a process for isolation and / or purification of proteins comprising an affinity chromatography, which is preferably performed on an affinity chromatographic matrix, to which a glycosaminoglycan, preferably heparin, is bound. Furthermore, the process of the present invention comprises a specific sequence of pre-chromatographic and purification steps.

The process of the present invention could be used for all proteins which comprise one or more amino acid regions enabling effective binding or increased affinity of the protein to the affinity chromatography matrix to which a glycosaminoglycan, in particular heparin, is bound. This amino acid region is preferably the region (Arg/Lys)XYZ(Arg/Lys). The process of the present invention could be therefore applied to the proteins which comprise one or more amino acid regions (Arg/Lys)XYZ(Arg/Lys) in the protein structure. In addition it can be applied for the proteins into which one or more of the amino acid regions (Arg/Lys)XYZ(Arg/Lys) are introduced, in particular to the proteins already bearing basic amino acid residues on the surface. Preferably, the process of the present invention applies for the members of TNF family ligands, in particular for TNF-alpha,TNF-alpha analogues and TNF-beta (limphotoxins). Most preferably, the protein is selected from the group consisting of TNF-alpha and TNF-alpha analogues.

The term '(Arg/Lys)' as used herein, refers to the use of either Lys or Arg in an amino acid sequence. The amino acid region (Arg/Lys)XYZ(Arg/Lys) is therefore selected from the group consisting of the amino acid regions ArgXYZArg, ArgXYZLys, LysXYZLys and LysXYZArg. In the sequence 'XYZ' as used herein, X, Y and Z refer to small, flexible, polar and / or charged amino acids, which are selected from the group comprising Ser, Gly, Ala, Thr, Pro, His, Lys, Arg, Gln and Asn that can be used in all possible combinations. The preferred amino acid regions comprised in the amino acid sequence of the proteins which are isolated and / or purified by the process of the present invention are ArgSerSerSerArg and ArgGlnHisProLys. The most preferred amino acid region is ArgSerSerSerArg.

The term 'TNF-alpha' as used herein, refers to the polypeptide of human TNF-alpha with native amino acid sequence. The biologically active TNF-alpha has a structure of a compact trimer with three equivalent N-terminal amino acid regions ArgSerSerSerArg.

The term 'covalently-linked trimer' as used herein, refers to a trimeric form of TNF-alpha or TNF-alpha analogue comprising at least two subunits, which are covalently linked, wherein the bond between the subunits is not a disulfide bond. This term is interchangeable with the term 'non-reducible trimers'.
The term 'TNF-alpha analogue' as used herein, refers to a polypeptide with certain mutations, deletions, and insertions or in general with any changes in amino acid sequence of TNF-alpha.
The term 'LK-805' as used herein refers to the TNF-alpha analogue, in which the mutation Glu107Lys is introduced. LK-805 was described in prior art in Novaković S *et* al Cytokine. 1997 9(8): 597-604.

The term 'elution' used herein refers to washing or extraction of the adsorbed material from the chromatographic column.
The term 'eluate' used herein refers to the solution, which is obtained by washing and extraction from the chromatographic column.
The term 'impurity' used herein refers to a substance which differs from the biologically active molecule of a protein, preferably of TNF-alpha or TNF-alpha analogues, such that the molecule is not biologically active. The impurity may also include further host cell substances such as proteins, DNAs, (lipo)polysaccharides etc., and additives which had been used in the preparation and processing of proteins, preferably TNF-alpha or TNF-alpha analogues. The term impurity includes also covalently linked trimers of TNF-alpha or a TNF-alpha analogue.
The impurity as indicated herein refers to a densitometric analysis of Commassie stained sodium dodecyl sulphate polyacrylamide electrophoresis (SDS-PAGE) gel using calibration curve in the same concentration range as impurity and using TNF as a reference material.

The process for isolation and / or purification of proteins of the present invention is particularly defined by comprising:
providing a mixture, which comprises a protein in the presence of an impurity, and
loading said mixture to an affinity chromatography matrix to which a glycosaminoglycan is bound.

The glycosaminoglycan is selected from the group consisting of heparin and heparan sulphate. In the preferred embodiment of the present invention an affinity chromatography matrix to which heparin is bound is used.

The process of the present invention preferably comprises the following steps:
- loading the mixture, which comprises the protein in the presence of an impurity, to an affinity chromatography matrix to which a glycosaminoglycan is bound,
- selective binding of the protein to the affinity chromatography matrix to which a glycosaminoglycan is bound, and
- eluting the protein from the matrix to which a glycosaminoglycan is bound to provide the protein.

In the preferred embodiment of the present invention the glycosaminoglycan is heparin.

In the preferred embodiment the present invention relates to process for isolation and / or purification of TNF-alpha and TNF-alpha analogues and is particularly defined by comprising:
providing a mixture, which comprises a protein in the presence of an impurity, and loading said mixture, to a affinity chromatography matrix to which a glycosaminoglycan is bound.

In particular the process of the preferred embodiment comprises the following steps:
- loading the mixture, which comprises TNF-alpha or a TNF-alpha analogue in the presence of an impurity, to an affinity chromatography matrix to which heparin is bound,
- selective binding of the protein to the affinity chromatography matrix to which heparin is bound, and
- eluting the protein from the affinity chromatography matrix to which heparin is bound to provide the protein.

The process for isolation and / or purification of proteins, in particular TNF-alpha or TNF-alpha analogues, of the present invention can, in addition to the affinity chromatography, further comprise one or more chromatographic steps, which can be performed before or after the affinity chromatography and are selected from the group consisting of anion exchange chromatography, size-exclusion chromatography, hydrophobic interaction chromatography, cation exchange chromatography and affinity chromatography. The purification steps can be applied in different combinations and / or in different order.

In the preferred embodiment of the present invention the process further comprises an anion exchange chromatography and a size-exclusion chromatography, wherein the anion exchange chromatography is preferably performed before the affinity chromatography and the size-exclusion chromatography is performed after the affinity chromatography.

In the preferred embodiment of the present invention, the process for isolation and / or purification of the proteins, preferably TNF-alpha and TNF-alpha analogues, comprises the following chromatographic steps:
a. anionic exchange chromatography and
b. affinity chromatography with an affinity chromatography matrix to which a glycosaminoglycan is bound, and
c. size-exclusion chromatography.
Preferably, the glycosaminoglycan is selected from the group of heparan sulphate and heparin. Most preferably, the glycosaminoglycan is heparin.
The chromatographic steps a., b., and c. are preferably performed in the order a., b. and c., but the order can be also different.

The process of the present invention results in the production of proteins, preferably TNF-alpha and / or TNF-alpha analogues, which could be suitable for clinical use in medicine.

The biologically active proteins, in particular TNF-alpha or TNF-alpha analogues obtained by the process for the isolation and / or purification of the present invention could be suitable for the preparation of pharmaceutical composition, which comprises the therapeutically effective amount of biologically active protein, preferably TNF-alpha or a TNF-alpha analogue and pharmaceutically acceptable auxiliary substances.
The possibility of maintaining the active form of protein obtained by the process of the present invention, preferably TNF-alpha or TNF-alpha analogues, in a short isolation and / or purification process contributes not only to a high yield, but also to a high purity and effectiveness of the biologically active protein, preferably TNF-alpha or TNF-alpha analogues, and the pharmaceutical composition containing it.
The term 'therapeutically effective amount' used herein refers to the amount of a biologically active protein, preferably TNF-alpha or a TNF-alpha analogue, which has the therapeutic effect of the biologically active protein, preferably TNF-alpha or a TNF-alpha analogue.
Suitable pharmaceutically acceptable auxiliary substances include suitable diluents, adjuvants and / or carriers useful in protein, in particular TNF-alpha or a TNF-alpha analogue, therapy.
Biologically active TNF-alpha or TNF-alpha analogues obtained by the process of the present invention, particularly when performing the additional steps of anionic exchange chromatography and size-exclusion chromatography, could be used for treatment of cancer and for preparation of medicaments for treatment of cancer.
It could be also used for preparation of medicaments for treatment and for treatment of all other illnesses, which are indicative for TNF-alpha or TNF-alpha analogues.
The pharmaceutical composition containing the pure and biologically active TNF-alpha or a TNF-alpha analogue obtained by the process of the invention could thus be administered, in a manner known to those skilled in the art, to patients in a therapeutically amount which is effective to treat the above mentioned diseases.

Preferred embodiments for performing the process for the isolation and / or purification of proteins according to the present invention are described in the following.

The process for the isolation and / or purification of proteins of the present invention preferably starts with the pre-chromatographic steps. The pre-chromatographic steps can comprise disruption of cells, precipitation of nucleic acids from the clear homogenate and precipitation of the protein fraction with ammonium sulphate (AS) or polyethyleneglycol.

The process of the present invention continues with the first chromatographic step, which can be an anionic exchange chromatography or a cationic exchange chromatography. Most preferably, an anionic exchange chromatography is applied.
The process begins with loading of the mixture, which comprises a protein in the presence of impurity, to the chromatographic matrix. The mixture (loading solution) is selected from the group consisting of a supernatant obtained directly after disruption of cells, supernatant after precipitation of nucleic acids from the clear homogenate, the solution of the protein precipitate in an appropriate buffer, the inclusion bodies solution or suspension (or mixture) in the presence of strong denaturating agents, such as 8 M urea or 6 M GndHCl, and solutions in the presence of denaturating concentrations of detergents (e.g. 1% sarcosyl, 2% sarcosyl or 1% sodium dodecyl sulfate), a solution which had been subjected to a previous renaturation, e.g. by dilution, dialysis, ultrafiltration or removal of denaturation agents/detergents. In case of the expression in secretory systems such as yeast, fungi or mammalian cell lines, the supernatant or concentrated supernatant or the culture medium can be used as a loading solution. The eluate resulting from the first elution from the anionic exchange / cationic exchange column can also be used as loading solution for loading on the anionic exchange / cationic exchange column once again. The eluate resulting from Heparin-Sepharose affinity chromatography can also be loaded onto anionic exchange / cationic exchange column.
Before loading to the column, the mixture, which comprises a protein in the presence of an impurity used as a loading solution, is preferably desalted to remove the salts that could interfere with the binding (the remaining ammonium sulphate or ammonium acetate or any other salts). The pH of the loading solution depends on the type of ionic exchange chromatography (anionic or cationic) used. Preferred pH of loading solution for anionic exchange chromatography is in the range from 7.5 to 8.5 in the case of TNF-alpha and TNF-alpha analogues with pl values, which are similar to TNF-alpha (pl is about 6.8) whereas pH in the range from 8.5 to 9.5 is preferred in the case of TNF-alpha analogues, which have higher pl values than TNF-alpha. The pH of the loading solution is preferably adjusted, e.g. by the addition of a NaOH solution or low concentrated acid (H₃PO₄...) solution or a high pH or low pH buffer solution. Buffer exchange can also be performed to achieve appropriate pH.
The first chromatographic step, which is preferably an anionic exchange chromatography, is used for elimination of most of *E. coli* proteins and other soluble components. It is also used as a capture step of nucleic acids, lipopolysaccharides and proteins derived from host cells, and for removal of ionic isomers of proteins, in particular of TNF-alpha or TNF-alpha analogues and changed (damaged) forms of proteins, in particular TNF-alpha or TNF-alpha analogues with altered pl values. The yield of this step is about 90 % with respect to the amount of target protein in the sample loaded onto the column.
Various anionic exchange chromatography supports can be used and may be selected from the group consisting of: DEAE-Sepharose CL-6B, DEAE-Sepharose FF, Q-Sepharose FF, Q-Sepharose HP, Q-Sepharose XL, DEAE-Sephacel, DEAE-Sephadex, QAE-Sephadex, DEAE-Toyopearl, QAE-Toyopearl, Mini-Q, Mono-Q, Mono-P, Source 15Q, Source 30Q, ANX-Sepharose etc. Preferably, the anionic exchange chromatography is performed on DEAE-Sepharose FF matrix.
In the case when cationic exchange chromatography is used, various cationic exchange chromatography supports can be used and may be selected from the group consisting of: CM-Sepharose CL-6B, CM-Sepharose FF, SP-Sepharose FF, SP-Sepharose HP, SP-Sepharose XL, CM-Sephadex, CM-Sephadex, CM-Toyopearl, SP-Toyopearl, Mini S, Mono S, Source 15S, Source 30S, TSK gel SP-5PW, TSK gel SP-5PW-Hr, Macro-Prep High S support, Macro-Prep S support, Macro-Prep CM support.

The salt concentration in the loading solution for anionic exchange chromatography is preferably low to enable the binding of the protein to the column. This is achieved with an appropriate method for buffer exchange. The binding of the protein to the column also depends on pH of the solution. Various buffers with the pH range from 7.5 to 8.5 can be used for loading and binding of proteins, in particular of TNF-alpha and TNF-alpha analogues, which have pl values similar to TNF-alpha, to the support for anionic exchange chromatography and may be selected from the group consisting of: phosphate, Tris/HCl, acetate, citrate, Tris/acetate, succinate, malonate, 2-(N-morfolinoethansulfonate) (MES) and other buffers. Preferably, phosphate buffer is used. Phosphate buffer can be used in the concentration range from 10 to 40 mM, preferably in the concentration range from 10 to 20 mM.
In case of isolation and / or purification of an TNF-alpha analogue with higher pl value than TNF-alpha, various buffers with the pH range from 8.5 to 9.5 can be used for loading and binding of the majority of E. coli proteins to the support. This pH prevents binding of the target protein to the support.
In the anionic exchange chromatography, the column loading is followed by washing of the column and the elution of the proteins from the column. The elution occurs due to increased ionic strength after the addition of high concentration of salt in buffer solution. Step gradient, linear gradient and a suitable combination of step gradient and linear gradient can be used for elution. Preferably, a suitable combination of step gradient and linear gradient are used.
Elution buffers, which can be used for washing and elution, may be selected from the group consisting of: phosphate, Tris/HCl, acetate, citrate, Tris/acetate, succinate, malonate, MES and other suitable buffers with addition of salts such as NaCl or KCl. Ionic strength and salt concentration, by which the elution is achieved, depends on the pH of the buffer solution. For the elution of TNF-alpha and TNF-alpha analogues, which have pl values similar to TNF-alpha, values salt concentration preferably in the range from 50 to 150 mM is used. The higher is pH of the buffer, the lower ionic strength is needed for the elution of the proteins from the column.
In the eluate, trimeric, biologically active TNF-alpha or TNF-alpha analogues with pl values similar to TNF-alpha (about 6.8), are obtained with a purity of greater than 80 %. In the case of TNF-alpha analogues with pl values higher than TNF-alpha, they are obtained in the flow-through fraction, in the trimeric, biologically active form, with a purity of greater than 80 %.

The second chromatographic step of the process of the present invention is preferably an affinity chromatography, preferably comprising affinity chromatography matrix to which a glycosaminoglycan is bound. The glycosaminoglycans are selected from the group consisting of heparin and heparan sulphate. In the preferred embodiment of the present invention heparin is bound to the affinity chromatography matrix of the affinity chromatography.

This step is an essential step for the final purity of the proteins obtained by the process of the present invention. In this step proteins, preferably TNF-alpha and TNF-alpha analogues bind to the affinity chromatography matrix more specifically, that is, with greater affinity than the other residual *E*. *coli* proteins
The affinity chromatography matrix is selected from the group consisting of Heparin-Sepharose 6 FF, Toyopearl AF-Heparin- 650M, etc. Preferably, the affinity chromatography matrix is Heparin-Sepharose matrix.

Mixture which comprises a protein in the presence of an impurity used as a loading solution for the second chromatographic step is preferably anionic chromatography eluate containing TNF-alpha or TNF-alpha analogue or anionic chromatography flow-through fraction containing TNF-alpha or TNF-alpha analogue.

This step in preferably performed at lower pH that allows significantly higher protein binding capacity. Therefore, prior to loading the mixture is preferably acidified to achieve pH 5.5 to 7.5. Preferably pH of the mixture is about pH 6.0. The mixture is acidified by preferably using 100 mM H₃PO₄ or any other diluted acid, which does not cause denaturation of the protein. In this phase, the binding capacity for TNF-alpha and TNF-alpha analogues with similar or higher pl values (in comparison to TNF-alpha) can be essentially increased by applying the sample in the buffer with pH 6.0. Various buffers with the pH range from 5.5 to 7.5, preferably about pH 6.0, can be used for loading and binding of TNF-alpha and TNF-alpha analogues with similar or higher pl values (in comparison to TNF-alpha) to the affinity chromatography matrix and may be selected from the group consisting of: phosphate, Tris/HCl, acetate, citrate, Tris/acetate, succinate, malonate, 2-(N-morfolinoethansulfonate) (MES) and other buffers. Preferably, phosphate buffer is used. Phosphate buffer can be used in the concentration range from 10 to 40 mM, preferably in the concentration range from 10 to 20 mM.
After binding to the matrix the process is continued by washing of the column and elution of proteins from the column. Elution can be performed by using suitable elution gradient to achieve maximal resolution whereby residual *E. coli* proteins are eluted first and at the end TNF-alpha or TNF-alpha analogue. Step gradient, linear gradient and a suitable combination of step and linear gradient can be used. Preferably, a suitable combination of step gradient and linear gradient are used.

Elution buffers, which can be used for washing and elution, may be selected from the group consisting of: phosphate, Tris/HCl, acetate, citrate, Tris/acetate, succinate, malonate, MES and other suitable buffers with addition of salts such as NaCl or KCl. Preferably, linear gradient from 0 to 500 mM NaCl is used with different slopes to achieve maximal resolution. Ionic strength and salt concentration, by which the elution is achieved, depends on the pH of the buffer solution. For the elution of TNF-alpha and TNF-alpha analogues, which have pl values similar to TNF-alpha, salt concentration in the range from 100 to 150 mM is used and for TNF-alpha analogues with pl values higher than TNF-alpha salt concentration in the range of 150 and 250 mM is used. The higher is pH of the buffer, the lower ionic strength is needed for the elution of the proteins from the column.

In the eluate biologically active TNF-alpha or TNF-alpha analogue is obtained with a purity of greater than 98%.

The third chromatographic step of the process of the present invention is preferably a size-exclusion chromatography. Size-exclusion chromatography is especially effective for removal of traces of dimers and higher aggregated forms of proteins, in particular of TNF-alpha or TNF-alpha analogues.
If desired, the eluate obtained from the affinity column can be loaded directly to the gel filtration column, without any additional intermediate steps being required. Preferably, prior to loading to size-exclusion chromatography column the eluate obtained from affinity column is concentrated to reduce the volume of the loaded sample. Different methods can be used: ultrafiltation, ammonium sulphate precipitation followed by dissolution in a small volume of appropriate buffer or any other appropriate method. Preferably ammonium sulphate precipitation is used.
Various size exclusion chromatography supports can be used and are selected from the group comprising: Sephacryl S-200HR, Sephacryl S-100HR, Superose 12, Superose 6, Superdex 75, Sephadex G-75, Sephadex G-100, Sephadex G-150, Sepharose 6B and CL-6B, Superdex75, Superdex 200, TSK gel G-2500PW, TSK gel G-3000 PW, Bio-Gel P-60, Bio-Gel P-100, Toyopearl HW-50, Toyopearl HW-55, Toyopearl HW65 etc. Preferably, the size-exclusion chromatography is performed on Superose 12.

A broad pH range of the loading solution for size-exclusion chromatography can be used. Loading of the solution, and elution of the protein can be performed by using the same buffer. Various buffers can be used and may be selected from the group consisting of phosphate, Tris and other suitable buffers, which can maintain pH in the range from 7.0 to 8.0. Preferably, phosphate buffers with pH from 7.0 to 8.0 are used. For loading the phosphate buffers can be preferably used in the concentration range from 20 to 100 mM, more preferably in the concentration range between 30 and 50 mM. The salt concentrations in the size-exclusion chromatography buffer can be in the range from 100 to 500 mM, preferably about 200 mM. Preferably, PBS buffer (Maniatis), containing 200 mM NaCl is used. In the eluate, biologically active, correctly folded protein, in particular TNF-alpha or TNF-alpha analogue is obtained with a purity of greater than 98 % and a full biological activity.

The following examples are given for the purpose of illustrating various embodiments of the invention and are not meant to limit the present invention in any fashion.

### Description of the drawings

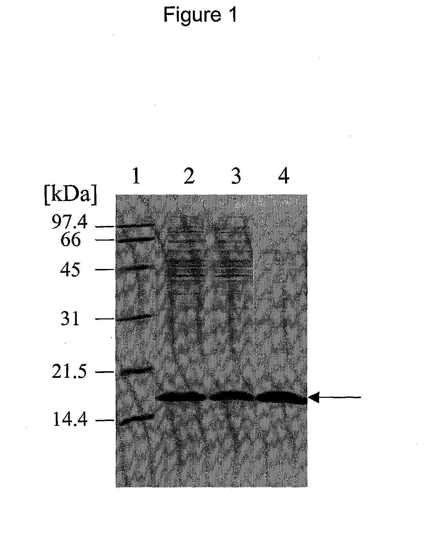

Figure 1 presents Coomassie-stained SDS-PAGE gel of the mixture comprising TNF-alpha after prechromatographic steps. The arrow shows the band comprising TNF-alpha.

### Legend

Lane 1: Molecular weight standard, Low Range (Bio Rad)
Lane 2: Supernatant after cell disruption, loading 5 µg
Lane 3: Supernatant after precipitation of nucleic acids with polyethyleneimine, loading 5 µg
Lane 4: Solution of ammonium sulphate precipitate, loading 5 µg

Figure 2 presents the Coomassie-stained SDS-PAGE gel of the final pure TNF-alpha. The arrow shows the band comprising TNF-alpha.

### Legend:

Lane 1: 1 µg of TNF-alpha
Lane 2: 2 µg of TNF-alpha
Lane 3: Molecular weight standard, Low Range (Bio Rad)
Lane 4: 5 µg of TNF-alpha

Figure 3 shows the chromatographic separation using the column HR10/10 with anionic support DEAE-Sepharose FF (Amersham Pharmacia Biotech). The chromatogram shows the absorbance change at 280 nm (A280) 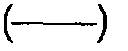 and the proportion of buffer P2 (-----) in dependence of time (min).
Peak A - flowthrough fraction containing *E. coli* proteins; peak B - bound proteins containing TNF-alpha and the remaining *E. coli* proteins.

Figure 4 shows the chromatographic separation using the column HR10/10, with Heparin Sepharose 6 FF support. The chromatogram shows the absorbance change at 280 nm (A280) 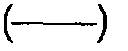 and the proportion of buffer P4 (-----) in dependence of time (min). The arrow marks the peak comprising TNF-alpha.

Figure 5 shows the chromatographic separation on the Superose 12 support. The chromatogram shows the absorbance change at 280 nm (A280) 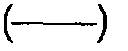 . The arrow marks the peak comprising TNF-alpha.

### Examples

### Example 1: Determination of binding capacity of TNF alpha at various pH on the Heparin Sepharose column

P1: 10 mM K-phosphate, pH 8.0
P2: 10 mM K-phosphate, pH 8.0, 1 M NaCl
P3: 10 mM K-phosphate, pH 6.0
P4: 10 mM K-phosphate, pH 6.0, 1M NaCl

### Binding capacity at pH 8.0

The column (Amersham Pharmacia Biotech, HR10/10, Vₘₐₜᵣᵢₓ = 8.30 ml) is equilibrated with the loading buffer P1 before loading. The column is loaded with 40 ml of the sample (DEAE-Sepharose eluate, pH 8.0; DEAE-eluate is prepared as described in Example 2). The total amount of proteins, loaded onto the column, is 9.5 mg, and the amount of TNF-alpha is 9 mg. Bound proteins are eluted with buffer P2. The flow rate is 2 ml/min, the 2-ml fractions are collected, and the whole process is performed at room temperature (22 °C). The flow-through fractions as well as bound fraction contain TNF-alpha. The amount of TNF-alpha in bound fraction is 6,6 mg and the amount in flow-through fraction is 2.2 mg. Approximately 0.8 mg of TNF-alpha is bound per 1 mL of Heparin Sepharose at pH 8.0 (maximal capacity at these conditions).

### Binding capacity at pH 6.0

The column (Amersham Pharmacia Biotech, HR10/10, Vₘₐₜᵣᵢₓ = 8.30 ml) is equilibrated with the loading buffer P3 before loading. The column is loaded with 7 ml of the sample (DEAE-eluate, acidified to pH 6.0; DEAE-eluate is prepared as described in Example 2). The total amount of proteins, loaded onto the column, is 13.5 mg, and the amount of TNF-alpha 12.5 mg. Bound proteins are eluted with buffer P4. The flow rate is 2 ml/min, the 2-ml fractions are collected, and the whole process is performed at room temperature (22 °C). The flow-through fraction contains no TNF-alpha. The amount of TNF-alpha in bound fraction is 12 mg. At least 1.4 mg of TNF-alpha is bound per 1 mL of Heparin Sepharose at pH 6.0 (minimal capacity at these conditions).

Binding capacity of TNF alpha to Heparin Sepharose is found to be significantly higher at pH 6 in comparison to pH 8. At least twice as much TNF alpha is bound at pH 6. This significantly improves the process economy and can be applied also for purification of TNF-alpha analogues.

### Example 2: Isolation and / or purification of biologically active TNF-alpha

### Preparation of loadinq sample (biomass):

The starting material is prepared using the following expression system: bacterial strain *E. coli,* BL21(DE3), plasmid: pCydcl containing properly inserted gene for TNF-alpha optimised for expression in E. coli (carrier plasmid BBG4, British Biotechnology) The expression plasmid pCydcl is prepared from commercially available plasmid pCYTEXP1 (Medac, Hamburg) by partial deletion of repressor gene cl857. Using pCydcl a constitutive expression of target protein at low temperature is achieved resulting into high accumulation of the protein (V. Menart et al. Biotech and Bioengineering, 83, No.2, 181-190, 2003). Protein is expressed in shaking flask cultures (total volume: 2L) at 30 °C. Weight of wet washed biomass is about 17 g (8,5 g/L).

### Pre-chromatographic steps

### Disruption of the cells

The biomass is resuspended in 70 ml (~ 4-fold volume) of buffer P50/30 (50 mM TRIS/HCI, 30 mM NaCl). The suspension is is homogenised using ultraturax PT3100 (Polytron). The cells are disrupted using the high-pressure homogeniser EmulsiFlex-C5 (Avestin) at working pressure 100000 kPa. After disruption, solid cell parts and insoluble portion of cellular proteins are removed by 30-minute centrifugation at 15000 rpm and 4°C.

### Precipitation of nucleic acids

In the supernatant after centrifugation nucleic acids are precipitated with polyethyleneimine. To the supernatant, 5 % polyethyleneimine is added slowly while mixing with a magnetic stirrer, to the final concentration of 0.1 %. The precipitate is removed by 30-minute centrifugation at 15000 rpm and 4°C.

### Precipitation of soluble proteins

In the supernatant after centrifugation soluble proteins are precipitated with ammonium sulphate. Solid ammonium sulphate is added slowly to the supernatant to 65% saturation (430 g/I), pH is simultaneously adjusted to the final pH between 7.0 and 8.0. The total amount of proteins in the suspension of ammonium sulphate precipitate is 495 mg, (~ 29 mg of proteins per 1 g of wet biomass). The suspension of ammonium sulphate precipitate is divided into aliquots each containing 50 mg of proteins. After 30-minute centrifugation at 15000 rpm and 4°C, the supernatant is poured-off, centrifuged again for 30 minutes under the same conditions and droplets of the supernatant are completely blotted by filter paper. The resulting ammonium sulphate precipitate is stored at 4°C.

### Determination of the content of TNF-alpha

By using a densiometric analysis of SDS-PAGE gels, stained with Coomassie blue, the content of TNF-alpha in the soluble fraction is determined after each pre-chromatographic step. The content of TNF-alpha in the supernatant after cell disruption is approx. 35 - 45 % TNF-alpha. After precipitation of nucleic acids TNF content is further increased to 40 - 50 %. Further enrichment of the sample is achieved by preparing an ammonium sulphate precipitate. The content of TNF-alpha in the solution of ammonium sulphate precipitate is 60 - 80 %.

### Chromatographic steps

### Buffers for chromatography

P1: 10 mM K-phosphate, pH 8.0
P2: 10 mM K-phosphate, pH 8.0, 1 M NaCl
P3: 10 mM K-phosphate, pH 6.0,
P4: 10 mM K-phosphate, pH 6.0, 1M NaCl
P5: PBS, pH 7.4, 200 mM NaCl
P6: PBS, pH 7.4, 500 mM NaCl

### Preparation of the sample for loadina onto DEAE-Sepharose

The aliquot of ammonium sulphate precipitate (total amount of proteins - 50 mg) is dissolved in 5 ml of buffer P1. Sample is desalted using the PD-10 column before loading onto the DEAE-Sepharose column to remove the remaining ammonium sulphate and other salts, interfering with binding to DEAE-Sepharose. The desalted sample is diluted to 10 ml with buffer P1, the final concentration is 5.3 mg/ml, determined by the Bradford method.

### 1^{st} chromatographic step

### Anionic chromatography, chromatographic matrix: DEAE-Sepharose

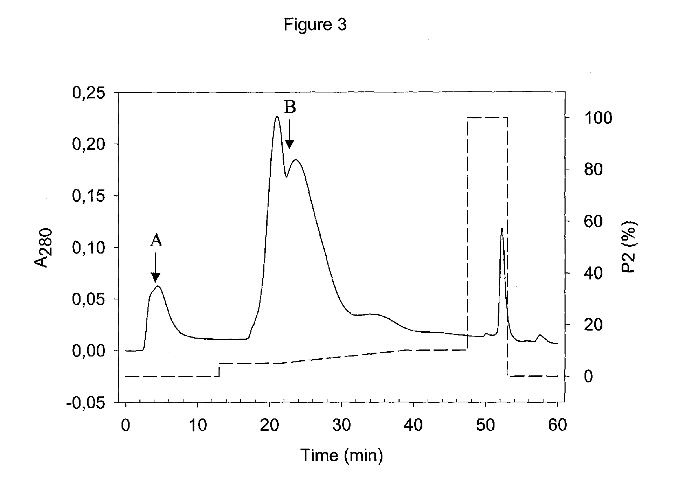

The column (Amersham Pharmacia Biotech, HR10/10, Vₘₐₜᵣᵢₓ = 7.85 ml) is loaded twice with ~ 5 ml of the sample. The total amount of proteins in the loading sample is - 50 mg, and the amount of TNF-alpha 37,5 mg. Prior to each loading the column is equilibrated with the starting buffer P1. The flow rate is 2 ml/min, the 2-ml fractions are collected, and the whole process is performed at room temperature (18-22 °C). The chromatogram is shown in Figure 3, the fractions of the principal peak (B) between 20^{th} and 30^{th} minute are pooled. The concentration of proteins in the pooled fractions after 1^{st} chromatographic step is 0.8 mg/ml, determined by the Bradford method, the total amount of proteins is - 36 mg of which - 34 mg TNF-alpha (purity ~ 94 %). Thus, the yield of 1^{st} chromatographic step is 90%.

### Acidification of the sample

The eluate from DEAE-Sepharose containing TNF-alpha is concentrated to ~ 20 ml in the Amicon cell with Millipore YM10 membrane. The final concentration is 1.7 mg/ml, determined by the Bradford method, the amount of proteins is - 34 mg, ~ 33 mg TNF-alpha. The yield of concentrating is ~ 97 %. Concentrated sample is acidified to pH 6.0 using 100 mM H₃PO₄ (100 µL of acid / ml of protein sample)

### 2^{nd} chromatographic step

### Affinity chromatography, chromatographic matrix: Heparin-Sepharose 6 Fast Flow

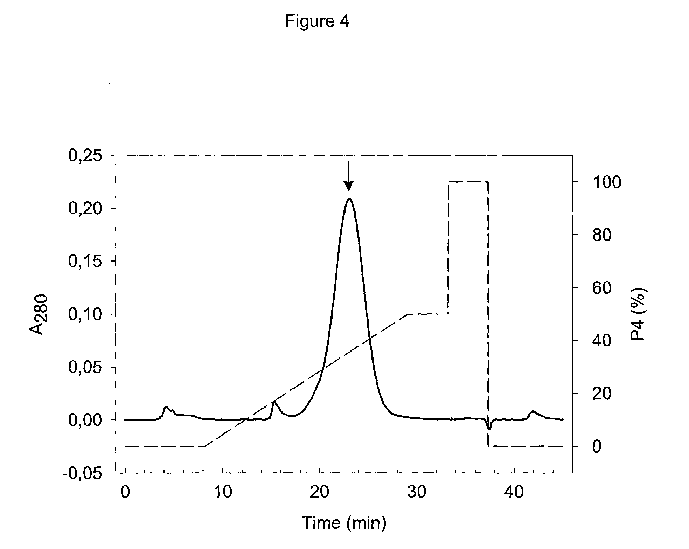

The column (Amersham Pharmacia Biotech, HR10/10, Vₘₐₜᵣᵢₓ = 8.60 ml) is equilibrated with the starting buffer P3 before each loading. The column is loaded three times with - 7 ml of the sample. The total amount of proteins, loaded onto the column, is 34 mg, and the amount of TNF-alpha 32 mg. The pH of both buffers, employed in this type of chromatography, is 6.0. The flow rate is 2 ml/min, the 2-ml fractions are collected, and the whole process is performed at room temperature (22 °C). The chromatogram is shown in Figure 4, the fractions of the principal peak between 20^{th} and 30^{th} minute are pooled. The concentration of proteins in the sample is determined by the Bradford method. The total amount of proteins after 2^{nd} chromatographic step is 22 mg, 21.5 mg of TNF-alpha (purity - 98 %). The yield of this step is - 67%, major loss occurs at cutting the chromatographic peak in order to choose the purest fractions for the last chromatographic step.

### Preparation of the sample prior to loading onto Superose 12

Prior to size-exclusion chromatography the sample is concentrated with the ammonium sulphate precipitation followed by the dissolution of the precipitate in a small volume of buffer P5. Solid ammonium sulphate is added slowly to the solution to 60% saturation (430 g/I). After 60-minute centrifugation at 15000 rpm and 4°C, the supernatant is poured off, centrifuged again for 30 minutes under the same conditions and droplets of the supernatant are completely blotted by filter paper. The ammonium sulphate precipitate is dissolved in 900 µl of buffer P5. The yield of precipitation and dissolution is - 70 %.

### 3^{rd} chromatographic step

### Size-exclusion chromatography, chromatographic matrix: Superose 12

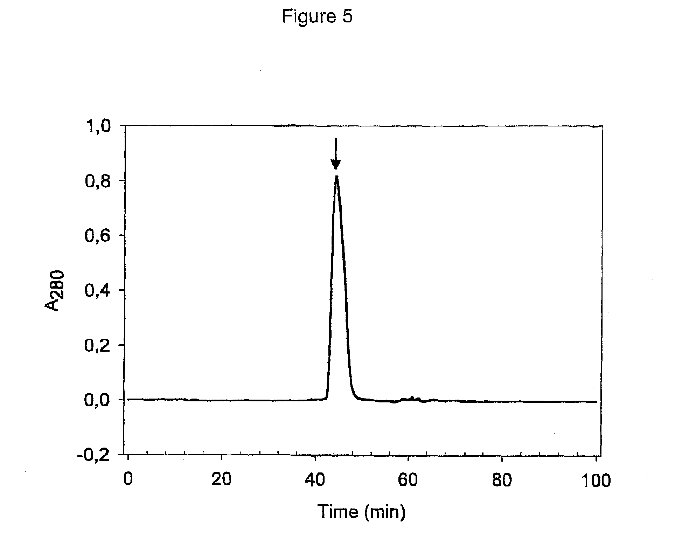

The column (Amersham Pharmacia Biotech, HR10/30, Vₘₐₜᵣᵢₓ = 23.8 ml, prepacked), equilibrated with buffer P5, is loaded three times with - 300 µl of the sample. The flow rate is 0.2 ml/min. Chromatogram of this chromatographic step is shown in Figure 5. The 0.2-ml fractions are collected and the fractions of the main peak between 66^{th} and 74^{th} minute are pooled. The concentration of TNF-alpha in the final sample is about 2.5 mg/ml, and the volume of a sample 4.8 ml. A suitable volume of 5 M NaCl is added to the sample to attain the final concentration of 0.5 M NaCl. The sample is diluted with buffer P6 to the concentration of TNF-alpha of 1 mg/ml. The final concentration of TNF-alpha, calculated from the absorbance measurement at 280 nm, is 1.03 mg/ml. Thus the total amount of TNF-alpha is 12.5 mg. The yield of the last chromatographic step is 80 %. The total yield of the isolation of TNF-alpha is 33 % (30 - 35 %).

### Characterisation of the purified protein

### SDS-PAGE

Covalently-linked trimer is seen in standard SDS-PAGE as an irreducible dimer.

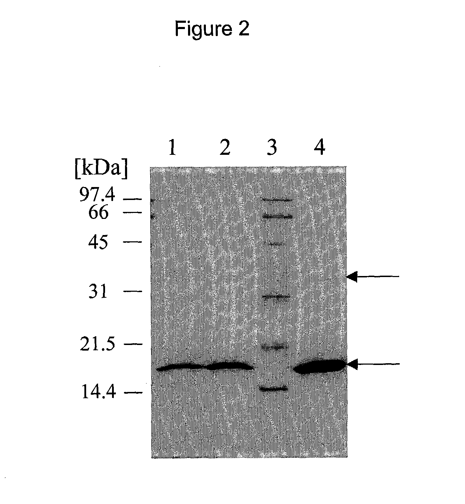

The SDS-PAGE is performed with loadings 1 µg, 2 µg and 5 µg of TNF-alpha. On the Coomassie stained gel, (Figure 2), at the 5 µg loading a trace of an irreducible dimer is visible beside the main band belonging to TNF-alpha monomer. The amount of irreducible dimer is less then 1 %. Bands belonging to the degradation products of TNF-alpha are not seen at this loading. Final purity is determined by using the densitometric measurement of Commassie blue stained SDS-PAGE gels. The amount of covalently-linked trimer is determined by comparing the band belonging to the irreducible dimer at 5 µg sample loading with the calibration curve prepared with the following standard (pure TNF) loadings: 0.05 µg, 0.1 µg, 0.25 µg and 0.5 µg. At protein loading of 1 µg per well and sensitive silver staining there are no other protein bands visible except the main spot belonging to TNF alpha monomer and less intensive irreducible dimer.

### Measurement of specific cytotoxycity of TNF-alpha and its analogues in vitro - Method for the immobilized cells

Specific cytotoxic activity is measured using the L-929 cell line (mouse fibroblasts) according to a modified procedure of Flick and Gifford (Flick, D.A., Gifford, G.E. 1984 68: 167-175). 2x104 cells in 100 l culture medium are seeded in each well of a microtiter plate and incubated for 24 hours (37C, 5 % CO2). After incubation the serial dilutions of internal TNF-alpha standard (prepared using WHO TNF-alpha standard 87/650) and TNF-alpha analogues are added into the wells in the presence of 2 g/ml actinomycin D. The plates are incubated again for 18 - 20 hours (37 C, 5 % CO2 ). The viable cells are then fixed with 2.5 % glutaraldehyde and stained with 0.5 % crystal violet in 20 % methanol. The plates are dried and 100 1 of 1 % SDS are added in each well. After 10 minutes of shaking at room temperature (- 25 °C), the optical density is measured at 570 nm. From the measured optical density the natural logarithm of the cell concentration is obtained. The specific cytotoxic activity of TNF-alpha and its analogues is determined by comparing the dilution of the standard and the samples yielding 50 % of maximal cytotoxicity *i*.*e*. 50% cells survived regarding the number of cells in the control wells without the protein (negative control) The specific cytotoxicity measured on L-929 cells is 3-4 x 10⁷ IU/mg.

### Example 3: Isolation and / or purification of LK-805 (E107K)

### Preparation of starting material (biomass)

The starting material is prepared using the following expression system: bacterial strain *E. coli*, BL21 (DE3), plasmid pCydcl with properly inserted gene for analogue LK-805. The expression plasmid pCydcl is prepared from commercially available plasmid pCYTEXP1 (Medac, Hamburg) by partial deletion of repressor gene cl857. Using pCydcl a constitutive expression of target protein at low temperature is achieved resulting into high accumulation of the protein (V. Menart et al. Biotech and Bioengineering, 83, No.2, 181-190, 2003).

Protein is expressed in shaking flask cultures (total volume: 2L) at 30 °C. Weight of wet washed biomass is ~ 17 g (8,5 g/L).

### Pre-chromatographic steps

### Disruption of the cells

The biomass is resuspended in 70 ml (~ 4-fold volume) of buffer P50/30 (50 mM TRIS/HCI, 30 mM NaCl). The suspension is homogenised using ultraturax PT3100 (Polytron). The cells are disrupted using the high-pressure homogeniser EmulsiFlex-C5 (Avestin) at working pressure 100000 kPa. After disruption, solid cell parts and insoluble portion of cellular proteins are removed by 30-minute centrifugation at 15000 rpm and 4°C.

### Precipitation of nucleic acids

In the supernatant after centrifugation nucleic acids are precipitated with polyethyleneimine. To the supernatant, 5% polyethyleneimine is added slowly while mixing with a magnetic stirrer, to the final concentration of 0.1%. The precipitate is removed by 30-minute centrifugation at 15000 rpm and 4°C.

### Precipitation of soluble proteins

In the supernatant after centrifugation soluble proteins are precipitated withammonium sulphate. Solid ammonium sulphate is added slowly to the supernatant to 65% saturation (430 g/L), pH is simultaneously adjusted to the final pH between 6.5 and 7.5. The total amount of proteins in the suspension of ammonium sulphate precipitate is 523 mg (~ 30 mg of proteins per 1 g of wet biomass). The suspension of ammonium sulphate precipitate is divided into aliquots each containing 50 mg of proteins. After 30-minute centrifugation at 15000 rpm and 4°C, the supernatant is poured-off, centrifuged again for 30 minutes under the same conditions and droplets of the supernatant are completely blotted by filter paper. The resulting ammonium sulphate precipitate is stored at 4°C.

### Determination of LK-805 content

The proportion of LK-805 in the soluble fraction is determined after each pre-chromatographic step using a densiometric analysis of Coomassie stained SDS-PAGE gels. In the supernatant after cell disruption there is - 50% of LK-805, and in the supernatant after precipitation of nucleic acids - 64 %, meaning that no target protein is lost with the removal of nucleic acids. Further enrichment of the sample is achieved by preparing an ammonium sulphate precipitate, the proportion of LK-805 in the solution of ammonium sulphate precipitate is - 66%.

### Chromatographic steps

### Buffers for chromatography

P1 a: 10 mM K-phosphate, pH 9.0
P2a: 10 mM K-phosphate, pH 9.0, 1 M NaCl
P3: 10 mM K-phosphate, pH 6.0
P4: 10 mM K-phosphate, pH 6.0, 1 M NaCl

### Preparation of the sample for loading on DEAE-Sepharose

The aliquot of ammonium sulphate precipitate (total amount of proteins ~100 mg) is dissolved in 10 ml of buffer P1a. By desalting in 50-ml Amicon cell (Millipore) using YM10 membrane, ammonium sulphate is removed. The concentration of proteins in the desalted sample is 7.55 mg/ml, determined by the Bradford method. Two 18 ml volumes of desalted sample are loaded onto the column.

### 1^{st} chromatographic step

### Anionic chromatography, chromatographic matrix: DEAE-Sepharose

The column (Amersham Pharmacia Biotech, HR10/10, Vₘₐₜᵣᵢₓ = 7.85 ml) is loaded with - 18 ml of the sample (- 90 mg). Before each loading the column is equilibrated against the starting buffer P1a. The flow rate is maintained at 2 ml/min, the 2-ml fractions are collected, and the whole process is performed at room temperature (22 °C). Although the sample is completely desalted and pH is above the pl value of the protein (pI_{LK-805} = 8.56), LK-805 does not bind to DEAE-Sepharose. However, most of *E. coli* proteins bind under these conditions. The unbound fractions are pooled. The concentration of proteins in the pooled fractions after 1^{st} chromatographic step is 2.38 mg/ml, determined by the Bradford method. The purity is between 80 and 85 %. The yield of 1^{st} chromatographic step with respect to LK-805 is 95 %.

### Acidification of the sample prior to loading on Heparin-Sepharose

Prior to loading on Heparin-Sepharose the sample is acidified with 100 mM H₃PO₄. Stepwise, during constant stirring, 3 ml of the acid per 30 ml of the sample are added (100 µL / 1 ml of sample).

### 2^{nd} chromatographic step

### Affinity chromatography, chromatographic matrix: Heparin-Sepharose 6 Fast Flow

The column (Amersham Pharmacia Biotech, HR10/10, Vₘₐₜᵣᵢₓ = 8.60 ml) is equilibrated with the starting buffer P3 before each loading. The column is loaded three times with - 7 ml of the sample. The pH of both buffers, employed in this type of chromatography, is 6.0, The flow rate is maintained at 2 ml/min, the 2-ml fractions are collected, and the whole process is performed at room temperature. A gradient NaCl from 1-500 mM is more gradual than for TNF-alpha - 10 VK instead of 5 VK.
Protein is eluted at -200-250 mM NaCl. The concentration of proteins in the sample is 1.2 mg/ml, determined by the Bradford method. The yield of the second chromatographic step is 60%.

## Claims

1. A process for the isolation and / or purification of proteins, which comprises:
providing a mixture which comprises a protein in the presence of an impurity, and
loading said mixture to an affinity chromatography matrix to which a glycosaminoglycan is bound.

2. The process according to claim 1, which comprises the following steps:
• loading said mixture, which comprises the protein in the presence of an impurity, to an affinity chromatography matrix to which a glycosaminoglycan is bound,
• selective binding of the protein to the affinity chromatography matrix to which a glycosaminoglycan is bound, and
• eluting the protein from the affinity chromatography matrix to which a glycosaminoglycan is bound to provide the protein.

3. The process according to claims 1 and 2, wherein prior to loading the mixture is acidified to achieve pH in the range from 5.5 to 7.5.

4. The process according to claim 3, wherein pH of the mixture is about 6.0.

5. The process according to claim 2 which further comprises one or more chromatographic steps, which can be performed before or after the process according to claim 2 and are selected from the group consisting of an anion exchange chromatography, size-exclusion chromatography, hydrophobic interaction chromatography, cation exchange chromatography and affinity chromatography.

6. The process according to claim 5, wherein the process comprises the following chromatographic steps:
a. anionic exchange chromatography, and
b. affinity chromatography with an affinity chromatography matrix to which glycosaminoglycan is bound, and
c. size-exclusion chromatography.

7. The process according to claims 1, 2 and 6, wherein the glycosaminoglycan is selected from the group consisting of heparan sulphate and heparin.

8. The process according to claim 7, wherein the glycosaminoglycan is heparin.

9. The process according to claims 1, 2 and 6, wherein the affinity chromatography matrix is Heparin Sepharose.

10. The process according to claim 6 wherein the anionic exchange chromatography is performed on DEAE-Sepharose FF matrix.

11. The process according to claim 6, wherein the size-exclusion chromatography is performed on Superose 12.

12. The process according any one of the preceding claims wherein the protein is selected from the group consisting of the proteins that comprise one or more amino acid regions (Arg/Lys)XYZ(Arg/Lys) in the protein structure and the proteins into which one or more of the amino sequence regions (Arg/Lys)XYZ(Ar g/Lys) are introduced.

13. The process according to claim 12, wherein the protein is selected from the group comprising TNF-alpha and TNF-alpha analogues.

14. The process according to claim 13, wherein the process comprises the following chromatographic steps:
a. anionic exchange chromatography, and
b. affinity chromatography with an affinity chromatography matrix to which glycosaminoglycan is bound, and
c. size-exclusion chromatography.

15. The process according to claim 14, wherein the selected glycosaminoglycan is heparin.
